# EUROPEAN PATENT APPLICATION

(11) **EP 2 581 447 A1**
(43) Date of publication of application: **17.04.2013**
(21) Application number: 11184940.2
(22) Date of filing: 12.10.2011
(51) Int. Cl.: C12N 15/10, C12Q 1/68

(54) **Selective isolation of a messenger RNA molecule having its cognate micro RNA molecules bound thereto**

(71) Applicant: Royal College of Surgeons in Ireland, Dublin 2 (IE); The Provost, Fellows, Foundation Scholars, & the other members of Board, of the College of the Holy & Undiv. Trinity of Queen Elizabeth near Dublin, Dublin 2 (IE)
(72) Inventor: Greene, Catherine, D15 Dublin (IE); Hassan, Tidi, D8 Dublin (IE); Smith, Stephen, D8 Dublin (IE); Mcelvaney, Noel, D2 Dublin (IE)
(74) Representative: Purdylucey Intellectual Property

(57) **Abstract**

A method of selectively isolating a target mRNA molecule having its cognate miRNA molecule(s) bound thereto and capable of being separated intact comprises the steps of treating a cell preparation with a crosslinking agent to reversibly fix mRNA molecules and their cognate miRNA molcules in a bound orientation and employing immobilised capture probe means to selectively bind the target mRNA and its bound miRNA. The capture probe means comprises one or more oligonucleotide probes which are complementary to an exposed single stranded loop portion of the target mRNA molecule, wherein the capture probe means selectively binds the target mRNA and its bound miRNA molecules. Furtherv steps include removing unbound material, eluting the isolated target mRNA and its bound miRNA from the support, and optionally quantitatively or qualitatively assaying the miRNA molecules bound to the target mRNA.

## Description

### Introduction

The invention provides a method of selectively isolating a mRNA molecule having its cognate miRNA molecules bound thereto. The invention also provides a kit suitable for performing the method of the invention.

Since the discovery of microRNAs (miRNA), which exhibit temporally and spatially controlled gene expression in plants and animals, as a major class of small interfering RNAs, much effort has gone into understanding how, when, and where miRNAs are produced and function in cells, tissues, and organisms. These highly conserved, ~21-mer RNAs regulate the expression of genes by binding to the 3'-untranslated regions (3-UTR) of specific mRNAs. Each miRNA is thought to regulate multiple genes and each gene can be regulated by multiple miRNAs. miRNAs have been shown to act as key regulators of processes as diverse as early metabolism, cell differentiation and cell death and are implicated in a number of diseases including chronic lymphocytic leukemia, colon and lung adenocarcinoma, cardiomyopathy and diabetes mellitus.

Target gene identification is challenging because in animals miRNAs bind to their target mRNAs by partial complementarity over a short sequence and suppression of an individual target gene is often undetectable. Although the rules of targeting are growing they are not yet completely understood. Bioinformatic analysis and computational algorithms are also developing but these can predict false positive miRNAs and miss *bona-fide* targets due to different algorithms placing variable weight on different features of mRNA:microRNA binding properties. Strategies have been developed whereby mRNAs and miRNAs can be co-immunoprecipitated based on ribonucleoprotein immunoprecipitation-microarray profiling (RIB-Chip) - a biochemical approach to isolate miRNAs that regulate specific groups of mRNAs that are functionally related. These approaches fail to isolate individual mRNA molecules.

An affinity capture technique is described in Vo et al (PNAS, Vol. 107, No: 45) which involves the generation of a hybrid mRNA comprising (a) a section of a 3'-UTR of a target mRNA, (b) MS2 mRNA, and (c) a GFP mRNA, transducing a test cell with the hybrid, lysing the test cell and capturing the hybrid on an affinity column using an MS2-binding protein as a capture probe, and then identifying any miRNA molecule bound to the 3'-UTR region. A disadvantage of this technique is that it only captures miRNA molecules that bind to the 3'-UTR region, and does not capture miRNA molecules that bind to other regions of the target mRNA. The technique is also laborious and involves the generation of a separate 3'UTR MS2 system for each mRNA. Further, as the technique employs only a fragment of the target mRNA, it is possible that the fragment will not adapt the natural three-dimensional configuration of the full length TARGET mRNA.

It is an object of the invention to overcome at least one of the above-referenced problems.

### Statements of Invention

The invention broadly provides a method of selectively isolating a single mRNA of interest with its miRNA molecule(s) bound within the RNA-inducing silencing complex (RISC). A cell is initially treated with a crosslinking agent, suitably with formaldehyde, to cross-link mRNA molecules along with their cognate miRNA molecules and associated RISC proteins. A pre-determined target mRNA can then be captured (with its miRNA and RNA-binding proteins intact) using one or more immobilized antisense oligonucleotide capture probes complementary to a single-stranded loop exposed within the target mRNA. In many cases, a single probe will be employed which will be complementary to a target sequence in the target mRNA which target sequence is unique to that mRNA and optionally all of its variants. In other cases where a unique target sequence of sufficient length is not available within an exposed single stranded loop portion of the target mRNA, selective binding of the target mRNA is achieved by means of a sequential capture process that employs at least two distinct capture probes each of which is complementary to a different target sequence, and wherein the presence of the two target sequences is unique to the target mRNA. Reversal of the cross-linking releases the selected mRNA and its miRNAs present in the RISC complexes which can then be analyzed for identification and/or quantification of the selected mRNA, and bound miRNAs, using various molecular biology tools such as RT-PCR, miRNA expression analysis based on a suitable microarray platform, or direct sequencing. The use of an exposed single stranded loop in the mRNA as a target for the capture probe provides for the formation of extremely strong hybrids between the capture probe and the target sequence, while also avoiding any disruption of miRNA-mRNA binding interactions.

Accordingly, the invention provides a method of selectively isolating a target mRNA molecule having its cognate miRNA molecule(s) bound thereto and capable of being separated intact, the method comprising the steps of:
- treating a cell preparation with a crosslinking agent to reversibly fix mRNA molecules and their cognate miRNA molcules in a bound orientation;
- employing immobilised capture probe means to selectively bind the target mRNA and its bound miRNA, in which the capture probe means comprises one or more oligonucleotide probes which are complementary to an exposed single stranded loop portion of the target mRNA molecule, wherein the capture probe means selectively binds the target mRNA and its bound miRNA molecules;
- removing unbound material; and
- eluting the isolated single mRNA and its bound miRNA from the support.

The method of the invention has a number of advantages over the affinity purification of Vo et al. First, the method is not limited to identifying only miRNAs that bind to the 3'UTR of a target mRNA. Further, the technique employs a full length mRNA rather than a fragment thereby ensuring that the target mRNA adopts its natural 3-dimensional conformation. Further, the technique is less laborious than the method of Vo et al which requires construction of different 3'UTR MS2 systems for each target mRNA.

In a preferred embodiment, the capture probe means comprises a single oligonucleotide capture probe having at least 15 contiguous nucleotides that is complementary to a target sequence located in an exposed single stranded loop portion of the target mRNA. Ideally the capture probe is an antisense molecule.

Suitably, the method includes a further step of reversing the cross-linking between the target mRNA and its cognate miRNA, and identifying the or each miRNA molecule bound to the target mRNA molecule.

Ideally, the oligonucleotide capture probe is an antisense molecule having 15-30 ribonucleotides

Preferably, the exposed single strand loop portion of the target mRNA is a sequence which is fully conserved amongst transcript variants of the target mRNA molecule.

Generally, the cell preparation is treated with the crosslinking agent and then lysed prior to incubation with the oligonucleotide capture probe.

Ideally, the crosslinking agent is formaldehyde.

The invention also provides an oligonucleotide probe of 15-35 contiguous nucleotides that is complementary to a target sequence of a target mRNA molecule, in which the target sequence comprises 15-35 contiguous nucleotides and is located in an exposed, single-stranded, loop portion of the mRNA molecule, and wherein the probe has specific binding affinity for the target sequence.

The invention also provides an oligonucleotide probe set comprising a plurality of oligonucleotide probes, each of which is optionally immobilised to a support, each oligonucleotide probe being at least a 10mer (ideally a 12-14mer) and being complementary to a different target sequence located in an exposed single stranded loop portion of a mRNA molecule, wherein the presence of the at least two target sequences is unique to the target mRNA. Thus, this aspect of the invention provides an oligonucleotide probe set suitable for selectively isolating a target mRNA molecule by means of a plurality of oligonucleotide capture probes each of which binds to a different target sequence located at an exposed single stranded loop portions of the target mRNA, and in which the presence of the at least two target sequences is unique to the target mRNA and optionally target mRNA variants.

Typically, the target sequence is present in most or all transcript variants of the target mRNA molecule.

Preferably, the oligonucleotide probe is an antisense molecule.

The invention also provides a kit suitable for performing a method of the invention, the kit comprising:
- an oligonucleotide capture probe comprising at least 15 contiguous nucleotides that is complementary to a target sequence located at exposed single strand loop portion of a target mRNA molecule;
- a support for the probe, in which the probe is immobilised to the support or capable of being immobilised to the support;
- optionally, a crosslinking agent suitable for crosslinking mRNA and their cognate miRNA molecules; and
- optionally, means for treating a cell preparation to lyse the cells.

Typically, the oligonucleotide probe is a 15-35mer which is complementary, ideally fully complementary, to the target sequence of the target mRNA molecule.

Suitably, the target sequence is present in most or all transcript variants of the target mRNA molecule.

Ideally, the support is a magnetic bead.

Preferably, the crosslinking agent is formaldehyde.

### Brief Description of the Figures

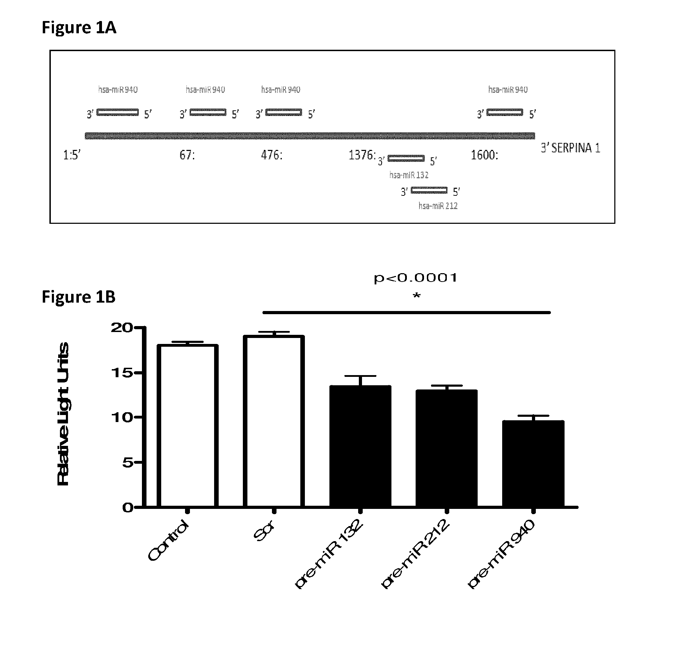

Figure 1: MiR-132, 212 and 940 directly target AAT mRNA. *A*, In-silico analysis of miRNA target prediction database shows predicted pairing between AAT 3'UTR and miR-132, 212 and 940. miR 940 was predicted to have 4 possible miRNA recognition elements (MRE) in the 3'UTR of the AAT mRNA while miR 132 and 212 were predicted to have only one B, Relative luciferase activity was measured in HEK 293 cells (1 x 10⁵ in triplicate) transiently transfected with pMIR-AAT-3'UTR and pRLSV40 and co-transfected with synthetic pre-miRs as indicated. *Firefly* luciferase activity was normalized to the *Renilla* luciferase activity. Data are represented as mean +/- SEM and were compared by *t*-test for Scr vs pre-miR; data is representative of three experiments.

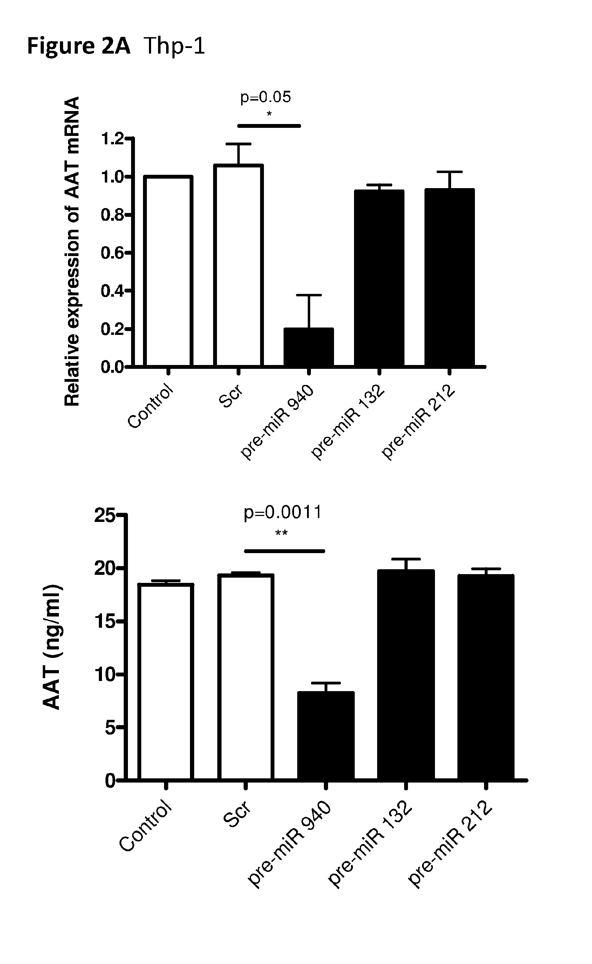

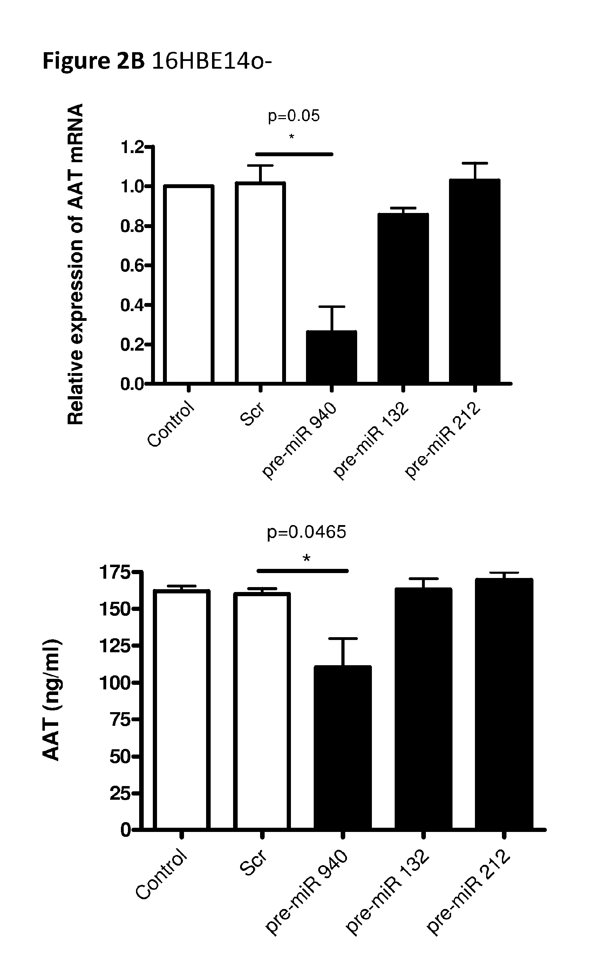

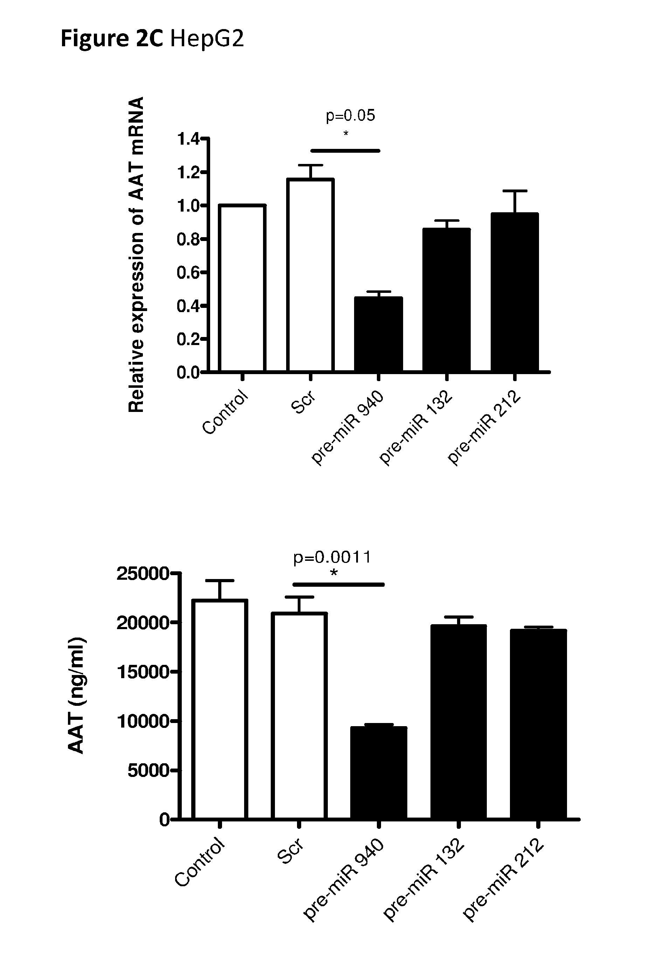

Figure 2: Effects of pre-miR 940, 132 and 212 overexpression on AAT mRNA (top) and protein (*below*) expression in *A*, THP-1, *B*, 16HBE14o-and *C*, HepG2. Cells (1×10⁵ in triplicate) were left untreated (control) or transfected with 30 nM of scrambled control (Scr) or synthetic pre-miRs as indicated. 24h post-transfection RNA was isolated and used in qRTPCR reactions with AAT and GAPDH primers. Relative AAT mRNA expression was quantified using the 2-^{ΔΔCt} method. 24 and 48h culture supernatants were assayed by ELISA to quantify AAT protein expression. Assays were performed in triplicate (n=3). Data were compared by *t*-test for Scr. vs pre-miRs.

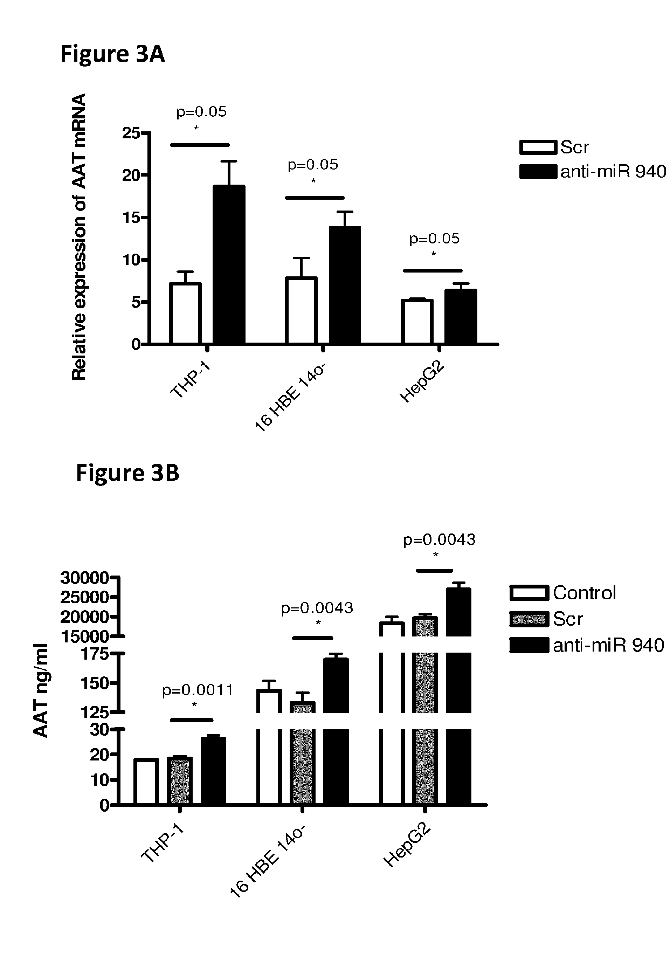

Figure 3: Effects of anti-miR 940 transfection on *A*, AAT mRNA and *B*, AAT protein expression in 3 cell lines. Cells (1x10⁵ in triplicate) were left untreated (control) or transfected with 30 nM of scrambled control (Scr) or synthetic anti-miR 940. 24h post-transfection RNA was isolated and used in qRTPCR reactions with AAT and GAPDH primers. Relative AAT mRNA expression was quantified using the 2-^{ΔΔCt} method. 24 and 48h culture supernatants were assayed by ELISA to quantify AAT protein expression. Assays were performed in triplicate (n=3). Assays were performed in triplicate (n=3). Data were compared by t-test for Scr. vs pre-miRs.

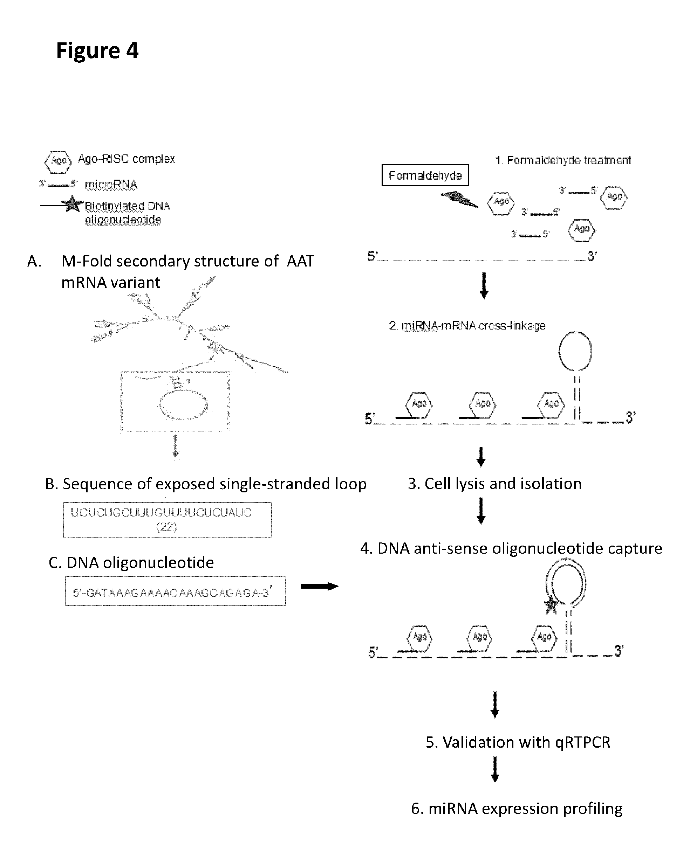

Figure 4: Strategy for single (AAT) mRNA:miRNA pull-down. Modelling the secondary structure of all transcript variants of AAT mRNA using M-fold A, the two most stringent conditions for each variant were selected. Based on these structures, an exposed single stranded region present in all variants B, were identified which is located between bases 2283 and 2304 of transcript variant 1 that can be used to design an anti-sense DNA oligonucleotide, *C*. *(1)* 3 cell lines (THP-1, 16HBE14o-and HepG2) were treated with formaldehyde to (*2*) cross-link miR-Ago-RISC complex with mRNA. *(3)* After cells are lysed, *(4)* the custom-designed biotinylated DNA oligonucleotides is used to capture only AAT mRNA:miRNA complexes with streptavidin beads. *(5)* Validation is performed to confirm the presence of AAT mRNA and miR-940, 132 and 212 and to rule out the evidence of other mRNAs and miRNAs once the mRNA:miRNA complexes are released. *(6)* The validated samples undergoes miRNA expression profiling.

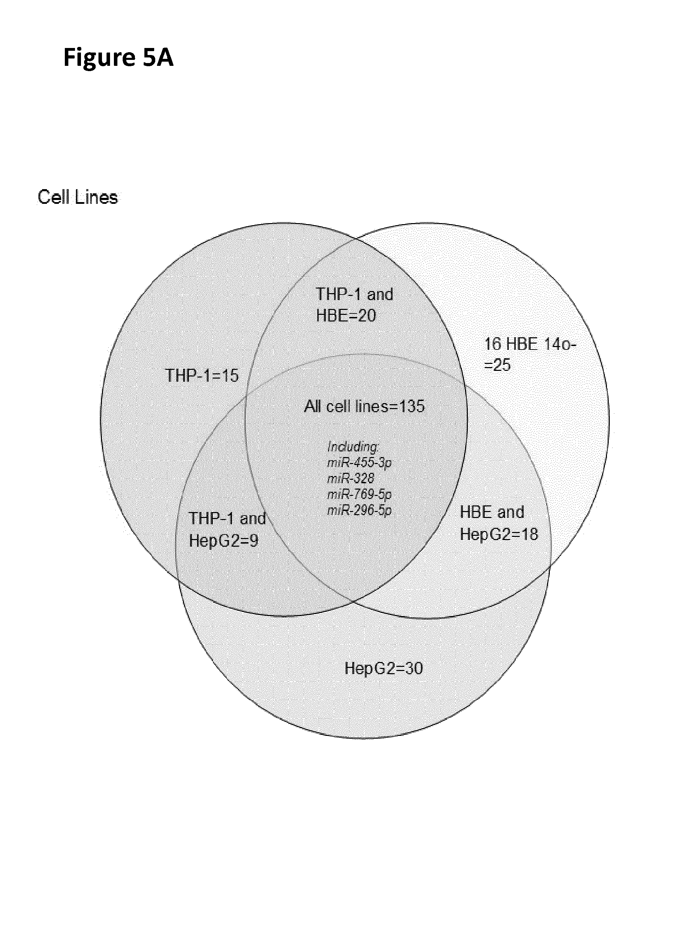

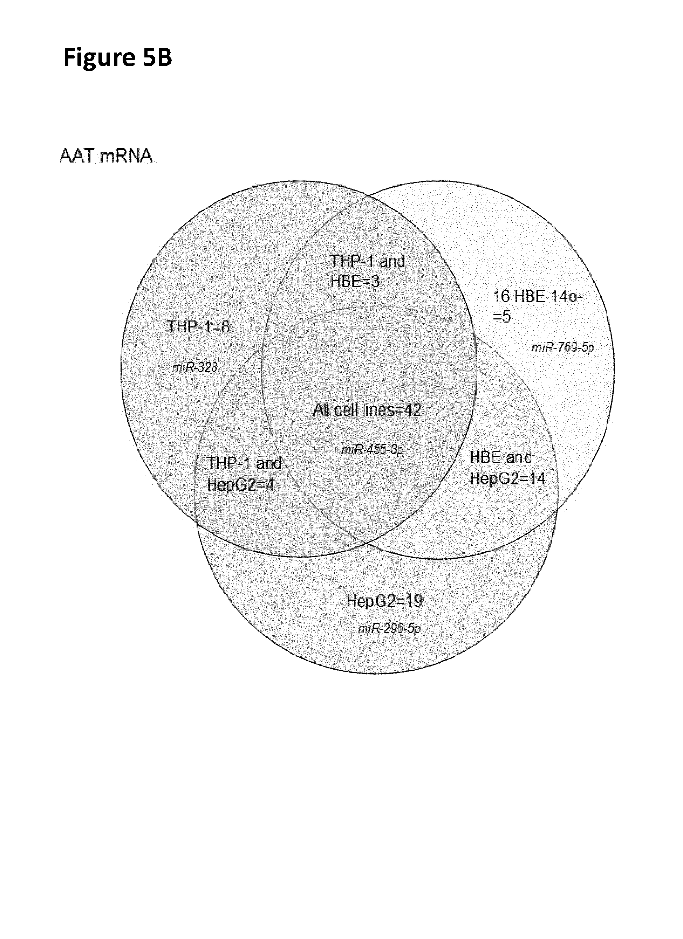

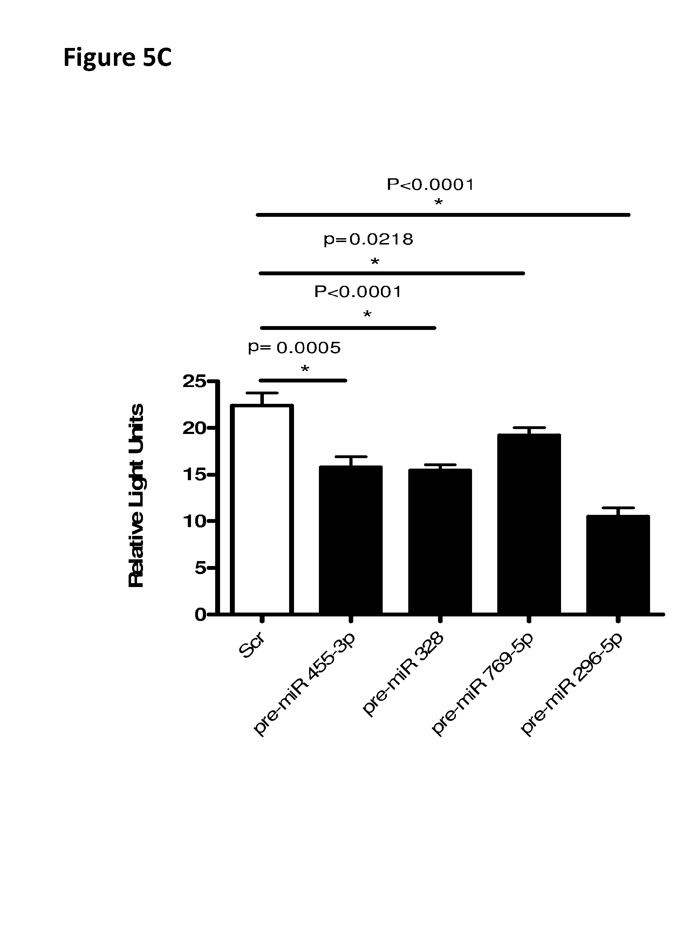

Figure 5: A, Total cell-specific miRNAs and B, AAT-specific miRNAs profiling using the nCounter Human miRNA Expression Kit (Nanostring Technologies) in THP-1, 16HBE14o-and HepG2. miRNAs were cross-referenced with target prediction databases (miRanda and TargetScan) for selection of 3'UTR-related miRNAs only. 4 miRNAs which were both cell and AAT specific were selected for relative luciferase activity in HEK 293 cells (1×10⁵ in triplicate). Cells transfected transiently with pMIR-AAT 3'UTR and pRLSV40 were co-transfected with their synthetic pre-miRs. Data are represented as mean +/- SEM and were compared with *t*-test.

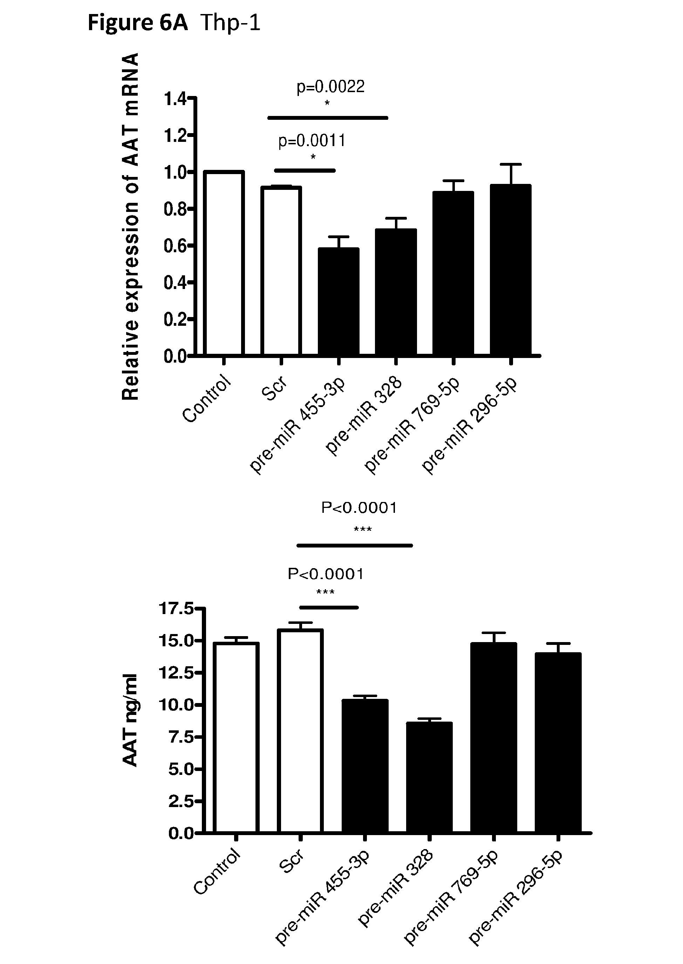

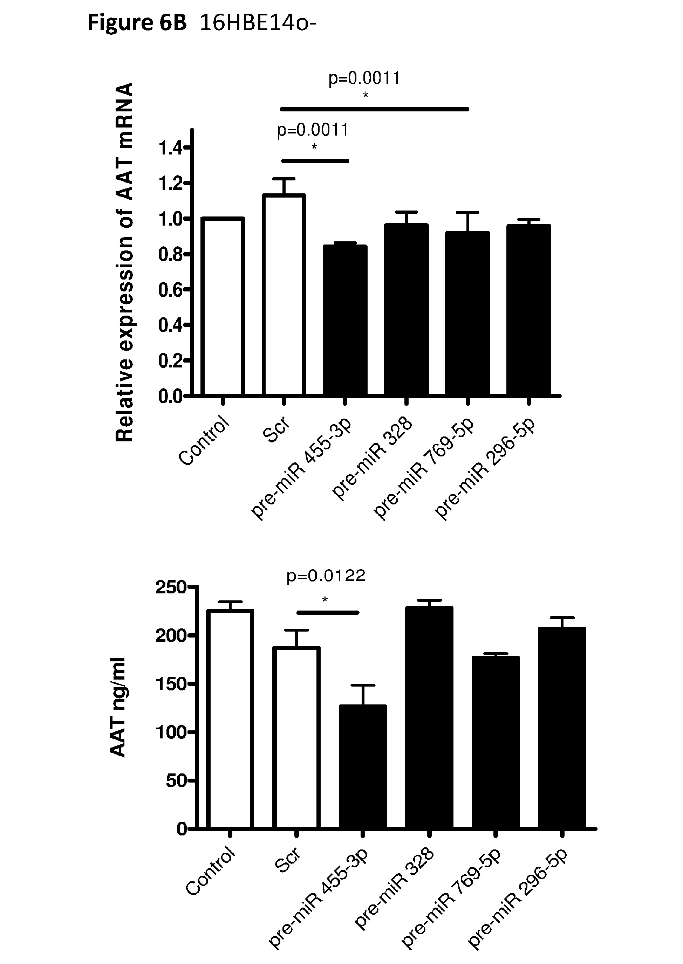

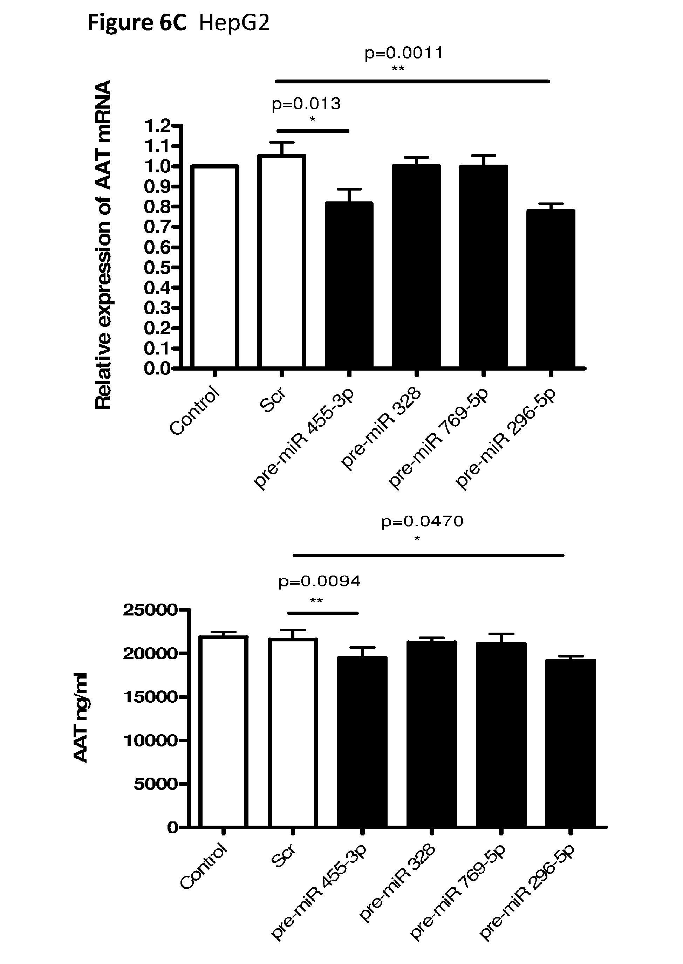

Figure 6: Effects of pre-miR 455-3p, 328, 769-5p and 296-5p overexpression on AAT mRNA (*top*) and protein (*below*) expression in *A*, THP-1, *B*, 16HBE14o-and *C*, HepG2. Cells (1×10⁵ in triplicate) were left untreated (control) or transfected with 30 nM of scrambled control (Scr) or synthetic pre-miRs as indicated. 24h post-transfection RNA was isolated and used in qRTPCR reactions with AAT and GAPDH primers. Relative AAT mRNA expression was quantified using the 2-^{ΔΔCt} method. 24 and 48h culture supernatants were assayed by ELISA to quantify AAT protein expression. Assays were performed in triplicate (n=3). Data were compared by t-test for Scr. vs pre-miRs. Note, miR-455-3p was expressed in all AAT-specific miRNAs assays, while miR-328, 769-5p and 296-5p were expressed in THP-1, 16HBE14o- and HepG2 respectively.

### Detailed Description of the Invention

Broadly, the invention provides a method for selectively isolating a specific mRNA molecule with its cognate miRNA molecule(s) bound thereto in an intact format. The invention involves treating biological material, for example a sample of cells, with a crosslinking agent such as formaldehyde to bind together the mRNA, its cognate miRNA, and associated RISC proteins. The cells are typically then lysed and the lysate is incubated with capture probe means capable of selectively binding the mRNA with its cognate miRNA molecules in a bound orientation. The capture probe means is ideally a single oligonucleotide probe having specific binding affinity for a target sequence in the target mRNA molecule located in an exposed single-stranded region of the mRNA. The probe is suitably at least a 15mer, and generally is a 15-35mer or a 20-30mer, and is complementary to the target sequence.

Should a target mRNA not have an exposed single stranded loop of 15-35 bases, this mRNA could nonetheless be captured effectively using 2 (or more) capture oligonucleotides, generally of less than 15 nucleotides in length, that ideally are 100% complementary to 2 (or more) exposed single-stranded loops present in the secondary structure of the target mRNA. Although the sequence of a 10-14mer exposed loop is unlikely to be unique to a specific target mRNA, when 2 (or more) such 10-14mers are employed to search the nucleotide database, only the target mRNA contains both (or all) sequences. Thus it is possible to isolate such a target mRNA using 2 (or more) capture oligonucleotides that independently can hybridise to a selection of mRNAs but, if used sequentially, their combined specificity can only capture the target mRNA of interest. Technically, if using 2 capture oligonucleotides, this would involve a 2-step sequential capture process. In the first affinity purification step one capture oligonucleotide is used and this step is followed by a second affinity purification using a second oligonucleotide. Thus, in an alternative embodiment (for example in cases where a target sequence of suitable length which is unique to the target mRNA cannot be located), the capture probe means comprises a plurality of oligonucleotide probes (for example, 2, 3, 4, 5, 6, or 7) each of which is complementary to a different target sequence located in an exposed single stranded loop portion of the target mRNA, and wherein the presence of the at least two target sequences is unique to the target mRNA.

In this specification, the term "complementary" as applied to the capture probe should be understood to mean that that the probe is at least 85%, and ideally at least 90%, complementary to the target sequence.

The method of the invention may be employed to selectively isolate a specific predetermined mRNA molecule with its cognate miRNA molecule(s) bound thereto in a reversible manner. This allows the miRNA molecules to be removed from the mRNA and assayed to, for example, identify and/or quantify the miRNA molecules. Thus, the invention relates to a method of identifying one or more miRNA regulatory molecules for a candidate mRNA molecule. The method of the invention enables the quantification of any given regulatory miRNA molecule for a specific mRNA target, and the quantity of the regulatory miRNA molecule bound to the target mRNA can then be correlated with other variables, for example the status of the cell (healthy/disease state/cell growth), and various environmental conditions (hypoxia, pH, etc). Thus, for example, the methods of the invention may be employed to assess the changes in levels of regulatory miRNA molecules for a specific target mRNA in a person in a specific disease state as compared to a healthy state. Further, the method of the invention may be employed to assess the changes in levels of regulatory miRNA molecules for a specific target mRNA in a person who is being treated with a specific drug.

The method of the invention is suitable for isolating from different sources a single mRNA molecule having its cognate miRNA molecules bound thereto. The different sources may be for example different organisms, different tissue, different cell types, the same or similar cell types under different conditions, for example cells with a disease phenotype and cells without a disease phenotype. The method of the invention is also suitable for isolating a single mRNA molecule (having its cognate miRNA molecule(s) bound thereto) from a single source but under different conditions, and determining the effect of different conditions on miRNA regulation of mRNA target molecules. The different conditions could be, for example, different environmental conditions such as temperature, light, pH, ionic concentration, oxygen availability, or different effector conditions, for example the effects of drugs, hormones, growth factors or the like. The method of the invention may be employed to compare miRNA binding to the same mRNA transcript in (1) different cell types in-vitro or in-vivo, (2) different species, (3) different disease states, (4) in response to treatments or therapeutics, or (5) at different stages of cell development. The method of the invention therefore facilitates investigations of what miRNA molecules are involved in regulation of specific proteins in specific cell types under specific conditions, and may be employed to understand how miRNA molecules affect protein expression in specific cell types in a disease and non-disease state.

The method of the invention involves preparation of a cell preparation, for example a cell lysate, in which the cell has been ideally pre-treated with an agent which fixes (crosslinks) each mRNA molecule, and its cognate miRNA molecule(s), in a bound orientation along with the protein RISC complex. In another embodiment, a cell peparation is first lysed and then treated with the cross-linking agent. The agent is typically formalehyde, although other crosslinking agents may be employed, for example UV light. Formaldehyde has been found to reversibly bind a mRNA, its cognate miRNA molecules, and its associated RISC complex, together leaving the miRNA and mRNA molecules intact and facilitating full release of the mRNA and miRNA molcules from the protein complex in an intact form.

As used herein, the term "oligonucleotide capture probe" means an oligonucleotide having at least 15 nucleotides and which has specific binding affinity for an exposed single stranded loop portion of the mRNA molecule (hereafter "target sequence"), and is antisense to the target sequence. Ideally, the probe is complementary to the target sequence. However, when the capture probe means comprises more than one oligo capture probe, then the probe may have as few as 10 or 12 nucleotides. The use of a single stranded exposed portion of the mRNA as the target for the probe facilitates the formation of exceptionally strong hydrids between the mRNA target sequence and the oligonucleotide probe. The exposed single stranded loop portion (target sequence) is typically a continuous portion of the mRNA molecule of at least 15, ribonucleotides, typically a 15-35mer, and ideally a 20-30mer, which is preferably present in most and ideally all transcript variants of the mRNA. The target sequence may be chosen by analysis of known transcript variants of the mRNA molecule of interest, and is preferably conserved amongst a plurality of transcript variants, and ideally is present in most or all known transcript variants. The term "known transcript variants" refers to transcript variants that are publicly available in PubMed. One suitable method of identifying a suitable target sequence is a mathematical prediction tool based on thermodynamic methods such as M-fold. For example, PubMed may be employed to identify all known transcript variants of the mRNA of interest. The secondary structure of each mRNA transcript variant can then be analysed using a mathematical prediction tool, mainly based on thermodynamic methods such as M-Fold (http://mfold.rna.albany.edu/?q=mfold/RNA-Folding-Form). Generally, the 2-3 most stringent conditions for each secondary structure are employed to locate exposed single-stranded regions which are present in all variants (i.e. 20-30 mers). A Basic Local Alignment Search Tool (BLAST) can then be employed to ensure that the sequence of the target sequence does not exist in other genes i.e. that it is unique to the mRNA.

The method of the invention also provides for separation of the mRNA:miRNA:RISC complex, to facilitate qualitative and/or quantitative assaying the mRNA or miRNA molecules. Separation can be achieved by treatment of the isolated material with a proteinase (to digest RNA-binding protein), optionally followed by treatment with a DNAase, followed by heat treatment to reverse the crosslinks between the mRNA and miRNA molecules. In a preferred embodiment, separation is provided a two step heat treatment comprising an initial step of heating the isolated material at a temperature of about 42 degrees C (+/- 5 degrees) in the presence of a proteinase (i.e. proteinase K) to allow digestion of cross-linked polypeptides followed by a second heat treatment at about 65 degrees C (+/- 10 or 5 degrees) to facilitate breakdown of the formaldehyde linkages.

The method of the invention involves selectively isolating a mRNA of interest with its cognate miRNA molecules bound thereto. The term "selectively isolating" should be understood to mean isolation of the specific mRNA of interest, preferably including most if not all of its transcript variants, from other different mRNAs present in the biological sample (i.e. lysed cell preparation). In one aspect, the method of isolation employs an oligonucleotide capture probe which has specific binding affinity for an exposed single stranded loop portion of the mRNA molecule of interest (target sequence). The term "specific binding affinity" should be understood to mean that the probe selectively binds to the target mRNA via binding to the target sequence and does not bind to non-target mRNA. Generally this is achieved by providing an antisense probe of at least 15 contiguous nucleotides that is complementary to the target sequence. If the target sequence is chosen such that it is present in all transcript variants of the target mRNA, then the capture probe will have specific binding affinity for the target mRNA and its transcript variants. The capture probe is immobilised to a support, and the support and immobilised capture probe is then incubated/reacted with for example the cell preparation to allow the mRNA:miRNA:RISC complex bind to the probe. Unbound and bound material is then suitably separated using conventional techniques. The bound material is then optionally released from the probe, and the binding of the mRNA and miRNA ideally reversed to release the isolated mRNA molecule and its cognate miRNA molecule(s) suitably in an intact format. The term "intact format" should be understood to mean that the molecules have not been cleaved or otherwise damaged and are capable of being assayed using conventional techniques.

The term "support" as employed herein refers to any support capable of supporting biologial molecules, for example a mobile solid phase or a stationary solid phase, and including the wells of a microtitre plate, a nitrocellulose membrane, or polymeric beads. The beads may for example be magnetic beads (i.e. paramagnetic or superparamagnetic beads) which are functionalised with the probe. For example, the beads may be coated with a biotin-binding partner (i.e. strepavidin) and the probe functionalised with biotin, allowing the probe and beads react by means of a streptavidin-biotin reaction. Other supports, and means of attaching the probe to the support, will be apparent to those skilled in the art.

The invention also provides a kit suitable for performing a method of the invention, the kit comprising:
- an oligonucleotide probe having specific binding affinity for an exposed single strand loop portion of the mRNA molecule;
- a support for the probe, in which the probe is immobilised to the support or capable of being immobilised to the support;
- optionally, a crosslinking solution (for example formaldehyde) suitable for crosslinking mRNA and their cognate miRNA molecules; and
- optionally, means for treating a cell preparation to lyse the cells.

The invention also provides an oligonucleotide probe immobilised to a support, wherein the immolilised probe has specific binding affinity for an exposed single strand loop portion of a target mRNA molecule (target sequence). The probe suitably is at least a 15mer, and preferably has 15-35 or 20-30 nucleotides. The probe is typically an antisense molecule, and is ideally complementary to the target sequence. The target sequence is typically a sequence of mRNA which is common to most or all transcript variants of the mRNA molecule and typically has 15-35 nucleotides.

The method of the invention provides a number of advantages over existing methods of analysing miRNA-mRNA interactions, including:
1. Is highly specific and can be custom-designed to detect miRNAs binding to any eukaryotic mRNA transcript.
2. Provides high-throughput identification of *bona fide* miRNAs targeting a specific mRNA
3. Eliminates false positive mRNA:miRNA interactions that can be predicted by *in silico* methods.
4. Bypasses initial bioinformatic screening
5. Can detect miRNAs bound anywhere within the entire mRNA transcript, not just the 3'UTR
6. Detects mRNAs, miRNAs and RISC complexes interaction in their natural cellular environment.

The methods of the invention find application in the following areas:
● Isolates a complex of the mRNA of interest together with its regulatory miRNAs and RISC.
● Can detect miRNAs bound anywhere within the entire mRNA transcript, not just the 3'UTR
● Can be used to compare miRNA binding to the same mRNA transcript in. for example (i) different cell types *in vitro* or *in vivo,* (ii) different species, (iii) different disease states, (iv) in response to different treatments or therapeutics, (v) at different stages of cell development (i.e. could be useful in stem cell biology).
● Provides an indication of the number of miRNA binding sites within a full length mRNA transcript
● Provides an indication of the strength of interaction between individual miRNAs and the target mRNA
● Can be used as an alternative experimental approach to validate pre-miR effects
● Can be used as an alternative experimental approach to validate anti-miR effects

### MATERIALS AND METHODS

### Cell culture and treatments

All cell lines were maintained in 37°C, humidified CO₂ incubator in appropriate media. THP-1 (human acute monocytic leukemia cell line), 16HBE14o- (human bronchial epithelial cell line), HepG2 (human hepatocellular liver carcinoma cell line) and HEK293 (human embryonic kidney cell line) were obtained from the European Collection of Cell Cultures (Salisbury. U.K). THP-1 and HepG2 were cultured in RPMI 1640 and DMEM (Sigma-Aldrich, St. Louis, MO) respectively while both 16HBE14o- and HEK293 were cultured in MEM (Sigma-Aldrich). All culture media contained 10% fetal calf serum (Gibco, Billings, MT) and 1% penicillin/streptomycin (Invitrogen, Carlsbad, CA) with the exception of HepG2 which was supplemented with L-glutamine and 0.1mM non-essential amino acids. Prior to treatment, cells were washed with serum free media.

### Quantitative assessment of mRNA and miRNA levels

RNA was isolated using TRI reagent (Sigma-Aldrich) according to the manufacturer's instructions. For quantification of mRNA, equal quantities of RNA were reverse transcribed into cDNA using the Quantitect Reverse Transcription kit (Qiagen, Valencia, CA). The resulting cDNA was template for quantitative real-time PCR. Oligonucleotide primers were synthesized (MWG, Biotech, Ebersberg) and quantitative PCR reactions performed in 20 ul containing 2 ul template cDNA, SYBR Green MasterMix (Roche, Basel, Switzerland) and 10 pmol of each primer for -AAT, GAPDH, β-actin, IL-8 and SLPI. MiRNAs expression was measured using Taqman miRNA assays (Applied Biosystems, Austin, TX) according to the manufacturer's instructions. Amplification for both mRNA and miRNA was performed on the Roche LC 480 Lightcycler in triplicate samples, including no-template controls. Relative expression of genes and miRNAs relative to GAPDH and miR-16 respectively were determined using the 2-^{ΔΔCt} method (PMID:11846609).

### AAT ELISA

Supernatants were recovered from THP-1, 16 HBE14o- and HepG2 cell lines. AAT protein concentration was determined by sandwich ELISA with specific Abs to AAT (ICN Biomedicals, Solon, OH) (PMID:21266225)

### Transfection of pre-miRs, anti-miRs and reporter plasmids

THP-1, 16HBE14o- and HepG2 (1 x 10⁵ in triplicate) were left non-transfected (control) or transiently transfected (24 and 48 hours) with either 30 nM of a scrambled control (Scr) or synthetic pre-miRs using Ribojuice (Novogen, Madison, WI) in OptiMEM reduced serum media (Life Technologies, Carlsbad, CA). RNA was isolated with TRI reagent (Sigma-Aldrich) while supernatants were recovered for ELISA. HEK293 cells ((1 x 10⁵ in triplicate) were transiently transfected with 250 ng pMIR-AAT-3'UTR (Origene, Rockville, MD) and 100ng of reference *Renilla* luciferase reporter plasmid pRLSV40 ((Promega, Madison, WI) with Genejuice (Novagen). Lysates were prepared and assayed for both firefly and *Renilla* luciferase using Luciferase assay system (Promega) and coelenterazine (Marker Gene Technologies, Eugene, OR). Firefly luciferase activity was normalized to the *Renilla* luciferase activity.

### DNA biotinylated oligonucleotide design

All of 11 mRNA transcript variants of AAT were modelled to their secondary structure using M-Fold ((http://mfold.rna.albany.edu/?q=mfold/RNA-Folding-Form). Two of the most thermodynamically stringent structure for each variant were selected and an exposed single-stranded region which was present in all variants was identified between bases 2283 and 2304 of transcript variant 1 (Figure 4a and b). Analysis with Basic Local Alignment Search Tool, or BLAST (http://blast.ncbi.nlm.nih.gov/Blast.cgi) showed the sequence to have 100% total coverage and be exclusive to all but only transcripts of AAT mRNA. An anti-sense DNA oligonucleotide sequence 5'-GATAAAGAAAACAAAGCAGAGA-3' (SEQUENCE ID NO: 1) with biotin modification was designed based on the canonical Watson-Crick RNA and DNA base-paring (Figure 4c).

### Formaldehyde cross-linking and cell lysis

Cells (0.5 x 10⁸ cells in triplicate) were treated with 37% formaldehyde (Sigma-Aldrich) for a final concentration of 1% and incubated at room-temperature for 15 minutes for RNA:RNA cross-linking (PMID:18265380). 0.2 M glycine was added to stop the cross-linking process and cells were washed with ice-cold Tris-buffered saline (TBS). Cell lysis was performed in lysis buffer (50mM HEPES pH 7.5, 140mM NaCl, 1mM EDTA, 1% Triton, 0.1% sodium deoxycholate) by using a FastPrep cell disrupter 4-5 times at speed 5.5 for 30 seconds. DNA removal was performed by adding RNAase-free DNAase (Qiagen) and this reaction was stopped with a final concentration of 20mM EDTA. Supernatants recovered following maximum speed microcentrifugation were used for mRNA:microRNA capture. All buffers were treated with 100x protease inhibitor (Roche, Mannheim, Germany) and RNAase inhibitor (Promega).

### Streptavidin bead preparation and miRNA:AAT mRNA pulldown

Immobilization of 2000 pmol/ml of biotinylated oligonucleotides with 10 mg streptavidin beads (Dynabeads M-280 Streptavidin) was performed according to manufacturer's instructions. Oligo-prepared beads were mixed with formaldehydetreated cell supernatants for 30 minutes at room temperature for adequate annealing. AAT mRNA:microRNA complex was eluted using the Dynal magnet. A final wash with 10mM TRIS-HCl (pH 7.5) was performed before heat treatment of 80_{°}C for 5 minutes to reverse the interaction between mRNA and Dynabeads. Cross-linked mRNAs and miRNAs were treated with proteinase K (Sigma-Aldrich) for digestion of RNA-binding proteins and remaining DNAase for 1 -hour 42_{°}C incubation. Incubation at 65_{°}C was performed to reverse the mRNA:miRNA cross-linkages. Validation was performed to confirm the presence of AAT mRNA and true target miRNAs and to rule out the evidence of other mRNAs and miRNAs by qRT-PCR and Taqman miRNA assay.

### Cell lines and AAT-specific miRNAs expression profiling

RNA isolated from non-treated cells and AAT-specific miRNAs from THP-1, 16HBE14o- and HepG2 cell lines were profiled commercially with the nCounter miRNA Expression Assay (Nanostring Technologies, Seattle WA). The samples were prepared with nCounter miRNA Sample Preparation Kit according to manufacturer's instructions. Raw data was normalized based on the relative number of positive control counts and adjusted for probe and background corrections for each miRNA as available in the nCounter Data Analysis Guidelines.

### Statistical Analysis

All analyses were performed using GraphPad PRISM 4.0 software package (San Diego, CA). Results are expressed as the mean +/- SEM and were compared by Student t test (non-parametric, one tailed; Mann-Whitney) or ANOVA as appropriate. Differences were considered significant at p values of less than 0.05.

### RESULTS

### In-silico prediction of miRNA targeting the AAT gene

In order to predict which miRNAs can potentially target the 3' UTR of AAT mRNA, all 11 transcript variants of AAT were analysed for putative miRNA binding sites using multiple individual databases including miRanda (http://www.microRNA.org), Targetscan (http://www.targetscan.org), microcosm (http://lwww.mirbase.org) and PicTar (http://pictar.mdc-berlin.de). These databases altogether predicted hundreds of miRNAs. miR-940 was chosen as this was predicted in multiple databases with 4 possible miRNA recognition elements (MRE) in the 3'UTR of the AAT mRNA; the fourth is situated at 3' end of the UTR, a location more predictive of a true target(PMID:17612493). This site also has a high miR-SVR score of -0.51 (PMID:20799968). Two other conserved miRNAs miR-132 and 212 were also selected which were predicted to have high miR-SVR score for their MRE sites for further study (Figure 1a).

AAT gene and protein expression were also quantified by qRT-PCR and ELISA respectively. AAT mRNA in THP-1, 16 HBE 140- and HepG2 cells with highest expression in the HepG2 > 16 HBE 14o- >THP-1. The cells produced 18 +/- 2, 148 +/-15, 17231 +/- 1092 ng/ml AAT respectively which was measured in cells' supernatants. miR 940, 132 and 212 were not differentially expressed in the three cell lines.

### AAT is a direct and functional target of miRNAs

To determine whether AAT is a molecular target of miR-940, 132 and 212, the effect of pre-miR-940, 132 and 212 on expression of a luciferase gene under the control of the AAT 3'UTR (pMiR-REPORT-AAT) in HEK293 cells was examined. This resulted in a significant decrease in luciferase gene expression from the reporter vector containing the AAT 3'UTR when compared with a scrambled control (Figure 1b), demonstrating direct molecular targeting by the three miRs (p < 0.0001). Controls performed with pre-miRs 126, 145 and 218 which are not predicted *in-silico* to regulate AAT showed no changes in luciferase gene expression (data not shown).

The effect of pre-miRs over-expression on the AAT gene was then assessed. 24 and 48 hours transfection of pre-miR 940, 132 and 212 into three cell lines THP-1, 16 HBE14o- and HepG2 cells resulted in a significant increase in all specific miRNAs expression, compared with non-transfected or scrambled-transfected cells as measured by miRNA Taqman assay (data not shown). qRT-PCR of the AAT gene showed significantly reduced expression in all three cells with pre-miR 940 overexpression only (p = 0.05) (Figure 2). Subsequent AAT ELISA on supernatants showed a reduction in AAT protein production and secretion compare with non-transfected cells or scrambled-transfected cells (Figure 2). There were no significant changes with AAT protein production with pre-miR 132 and 212 transfection. Combined transfections with more than one pre-miR that included pre-miR 940 only showed a reduction of both the AAT gene and protein (data not shown).

Whether anti-miR-940 functionally increases AAT gene and protein production was also assessed. Cells transfected with anti-miR 940 resulted in significant increase in AAT gene expression (Figure 3a) and protein production (Figure 3b).

### Validation of AAT-specific miRNA isolation technique

To validate the method of the miRNA:mRNA complexes pull-down (Figure 4), qRT-PCR was performed with AAT, GAPDH, B-actin and SLPI primers. Besides AAT, there was no evidence of other gene transcripts present. miRNA Taqman assay was also performed to confirm the presence of miR-940, 132 and 212 and to rule out the presence of miR-126, 145 and 218.

### miRNA expression profiling and testing AAT and cell-specific miRNAs

Profiling total and AAT-specific miRNA from THP-1, 16 HBE 14o- and HepG2 cells to examine the expression of 731 different human miRNAs was performed using NanoString Technologies miRNA Expression Kit. Appreciable target detection (normalized data after probe and background correction >1) occurred for 252 miRNAs across all 3 cell lines (135 in all cell lines; 15, 25 and 30 exclusively in THP-1, 16 HBE 14o- and HepG2 respectively; 20, 9 and 18 in two of the cell lines) (Figure 5a).

When AAT-specific miRNAs were profilled, 42, 8, 5 and 19 AAT-specific miRNAs were detected in all three cell lines, THP-1, 16HBE14o- and HepG2 cells respectively (Figure 4b). These miRNAs were cross-referenced with the target prediction databases miRanda and TargetScan to identify 3'UTR miRNAs only. Of these, 4 miRNAs with good miR-SVR scores which were both cell and AAT-specific were selected including miR-455-3p expressed in all cell lines, miR-328, miR 769-5p and miR-295-5p expressed exclusively in THP-1, 16HBE 14o- and HepG2 respectively. Co-transfection with pre-miRs resulted in a significant decrease in luciferase gene expression from the reporter vector containing the AAT 3'UTR (Figure 5C) when compared with non-transfected and scrambled-transfected cells, demonstrating direct molecular targeting by miRNAs isolated from AAT mRNA:miRNA complexes.

### Functional effects of AAT-specific pre-miRs

To determine whether the AAT specific miRNA have cell-specific effects on AAT expression, the selected miRNAs were overexpressed in three cell lines using pre-miRs transfection. miR 455-3p is expressed in all three cell lines. Overexpression of pre-miR 455-3p significantly decreased both AAT mRNA and protein expression in THP-1, 16 HBE 14o- and HepG2 cells (Figure 6). miR 328 targets AAT and is expressed uniquely in THP-1. miR-328 overexpression resulted in a significant reduction of AAT mRNA and protein in THP-1 cells only. Similar results were also seen with miR-769-5p in 16HBE14o- and miR-296-5p in HepG2.

The invention is not limited to the embodiments hereinbefore described which may be varied in construction and detail without departing from the spirit of the invention.

Organization Applicant
---------------------
      Street : College Green
      City : Dublin
      State :
      Country : Ireland
      PostalCode :
      PhoneNumber :
      FaxNumber :
      EmailAddress :
<110> OrganizationName : The Provost, Fellows, Foundation Scholars, and
other Members of Board, of the College of the Holy and Undivided Trinity
of Queen Elizabeth, near Dublin

Organization Applicant
---------------------
      Street : 121 St Stephens Green
      City : Dublin
      State :
      Country : Ireland
      PostalCode : D2
      PhoneNumber :
      FaxNumber :
      EmailAddress :
<110> OrganizationName : Royal College of Surgeons in Ireland

Application Project
-------------------
<120> Title : SELECTIVE ISOLATION OF A  MESSENGER RNA MOLECULE HAVING ITS

       COGNATE MICRO RNA MOLECULES BOUND THERETO
<130> AppFileReference : P10756EP00
<140> CurrentAppNumber :
<141> CurrentFilingDate : ____-__-__

Sequence
--------
<213> OrganismName : Synthetic oligonucleotide
<400> PreSequenceString :
gataaagaaa acaaagcaga ga                                              22
<212> Type : DNA
<211> Length : 22
      SequenceName : SEQUENCE ID NO: 1
      SequenceDescription :

## Claims

1. A method of selectively isolating a target mRNA molecule having its cognate miRNA molecule(s) bound thereto and capable of being separated intact, the method comprising the steps of:
- treating a cell preparation with a crosslinking agent to reversibly fix mRNA molecules and their cognate miRNA molcules in a bound orientation;
- employing immobilised capture probe means to selectively bind the target mRNA and its bound miRNA, in which the capture probe means comprises one or more oligonucleotide probes which are complementary to an exposed single stranded loop portion of the target mRNA molecule, wherein the capture probe means selectively binds the target mRNA and its bound miRNA molecules;
- removing unbound material; and
- eluting the isolated target mRNA and its bound miRNA from the support.

2. A method as claimed in Claim 1 including a further step of reversing the cross-linking between the target mRNA and its cognate miRNA, and qualitatively or quantitatively assaying the or each miRNA molecule bound to the target mRNA molecule.

3. A method as claimed in 1 or 2 in which the oligonucleotide capture probe is an antisense molecule having 15-30 ribonucleotides that is complementary to a target sequence located at the exposed single strand loop portion of the target mRNA molecule.

4. A method as claimed in any preceding Claim in which the exposed single strand loop portion of the target mRNA is a sequence which is fully conserved amongst transcript variants of the target mRNA molecule.

5. A method as claimed in any Claim 1 in which the immobilised capture probe means comprises at least two oligonucleotide capture probes each of which is at least a 12mer and is complementary to a different target sequence located at the exposed single stranded loop portion of the target mRNA, wherein the at least two target sequences is unique to the target mRNA.

6. A method as claimed in any preceding Claim in which the cell preparation is treated with the crosslinking agent and then lysed prior to incubation with the oligonucleotide capture probe.

7. A method as claimed in any preceding Claim in which the crosslinking agent is formaldehyde.

8. An oligonucleotide probe of 15-35 contiguous nucleotides that is complementary to a target sequence of a target mRNA molecule, in which the target sequence comprises 15-35 contiguous nucleotides and is located in an exposed, single-stranded, loop portion of the mRNA molecule, and wherein the probe has specific binding affinity for the target sequence.

9. An oligunucleotide probe as claimed in Claim 8 in which the target sequence is present in most or all transcript variants of the target mRNA molecule.

10. An oligonucleotide probe set comprising a plurality of oligonucleotide probes, each of which is optionally immobilised to a support, each oligonucleotide probe being at least a 10mer and being complementary to a different target sequence located in an exposed single stranded loop portion of a target mRNA molecule, wherein the presence of the at least two target sequences is unique to the target mRNA.

11. An oligonucleotide probe of claim 8 or 9, or an oligonucleotide probe set of Claim 10, in which the or each oligonucleotide probe is immobilised to a support.

12. A kit suitable for performing a method of any of Claims 1 to 4, the kit comprising:
- an oligonucleotide capture probe comprising at least 15 contiguous nucleotides that is complementary to a target sequence located at exposed single strand loop portion of a target mRNA molecule;
- a support for the probe, in which the probe is immobilised to the support or capable of being immobilised to the support;
- optionally, a crosslinking agent suitable for crosslinking mRNA and their cognate miRNA molecules; and
- optionally, means for treating a cell preparation to lyse the cells.

13. A kit as claimed in Claim 10 in which the target sequence is present in most or all transcript variants of the target mRNA molecule.

14. A kit as claimed in any of Claims 12 or 13 in which the support is a magnetic bead.

15. A kit as claimed in any of Claims 12 to 14 in which the crosslinking agent is formaldehyde.
